# NEUE EUROPÄISCHE PATENTSCHRIFT

(11) **EP 2 497 507 B2**
(45) Veröffentlichungstag und Bekanntmachung des Hinweises auf die Entscheidung über den Einspruch: **14.09.2022**
(45) Hinweis auf die Patenterteilung: 19.02.2014
(21) Anmeldenummer: 11157528.8
(22) Anmeldetag: 09.03.2011
(51) Int. Cl.: A61M 1/16

(54) **Dialysevorrichtung**
Dialysis device
Dispositif de dialyse

(43) Veröffentlichungstag der Anmeldung: 12.09.2012
(73) Patentinhaber: B. Braun Avitum AG, 34212 Melsungen (DE)
(72) Erfinder: Ritter, Kai-Uwe, 34212, Melsungen (DE)
(74) Vertreter: Winter, Brandl - Partnerschaft mbB

(56) Entgegenhaltungen:
- EP-A1- 0 428 009
- EP-A1- 2 595 670
- US-A- 5 861 555
- US-A1- 2003 212 314

## Beschreibung

Die vorliegende Erfindung befasst sich mit einer Dialysevorrichtung gemäß dem Oberbegriff des Patentanspruchs 1.

Die Dialyse ist ein Blutreinigungsverfahren, das bei Nierenversagen als Ersatzverfahren zum Einsatz kommt. Die Dialyse ist neben der Nierentransplantation die wichtigste Nierenersatztherapie bei chronischem Nierenversagen und eine der möglichen Behandlungsalternativen bei akutem Nierenversagen. Ziel der Dialyse ist es, die Konzentration harnpflichtiger bzw. pathogener Substanzen im Blut bzw. im Körper des Patienten unterhalb einer toxischen Grenze zu halten.

Blutreinigende Therapieverfahren basieren auf den physikalischen Grundprinzipien Diffusion, Osmose und Konvektion und teilweise auch auf Adsorption. Die unterschiedlichen Verfahren der Dialyse basieren dabei meist auf einem Stoffaustausch über spezielle, semipermeable Membranen, die im Folgenden als Dialysemembranen bezeichnet werden. Die Dialyse be- wirkt unter anderem die Reinigung des Blutes, den Entzug von Wasser aus dem Blutkreislauf und das Hinzufügen von Elektrolyten zum Blut. Bei der Dialyse befindet sich auf der einen Seite der Dialysemembran Blut oder Blutplasma und auf der anderen Seite der Membran eine Dialyselösung bzw. eine Dialysierflüssigkeit. Je nach Beschaffenheit der Membran diffundieren unterschiedliche Substanzen durch die Membran vom Blut in die Dialysierflüssigkeit und werden so dem Blutkreislauf entzogen. Gleichzeitig können andere Substanzen, beispielsweise Elektrolyte, auch von der Dialysierflüssigkeit in das Blut diffundieren. Eine der entscheidenden Parameter ist dabei die Beschaffenheit der Membran und insbesondere deren Porengröße, durch die wesentlich bestimmt wird, welche Substanzen bei der Dialyse aus dem Blut entfernt werden. Bei Verwendung solcher semipermeabler (teildurchlässiger) Membranen spricht man daher auch von selektiver Diffusion. Während die Membran im Wesentlichen die Art bzw. die Größe der prinzipiell austauschbaren Substanzen bestimmt, wird die Diffusionsgeschwindigkeit und damit der tatsächliche Austausch im Wesentlichen durch die Konzentrationsunterschiede der jeweiligen Substanzen auf den unterschiedlichen Seiten der Membran bestimmt. Die zu entfernenden Substanzen werden grob in niedrig-, mittel-, und hochmolekulare Substanzen klassifiziert. Durch Osmose bzw. eine künstlich zwischen den unterschiedlichen Seiten der Dialysemembran erzeugte Druckdifferenz kann darüber hinaus der Flüssigkeitshaushalt des Patienten kontrolliert werden, indem dem zu reinigenden Blut Flüssigkeit entzogen wird. Zusätzlich können durch spezielle Zusätze in der Dialysierflüssigkeit der Säure-Basenstatus und die Elektrolytzusammensetzung des Blutes beeinflusst und reguliert werden.

Als Dialysemembranen werden zum einen künstliche bzw. technische Membranen verwendet, zum anderen aber auch körpereigene, physiologische Membranen. Es wird also bei der Dialyse zwischen extrakorporalen Verfahren, die künstliche Membranen außerhalb des Körpers verwenden und intrakorporalen Verfahren, die innerhalb des Körpers durchgeführt werden und dabei körpereigene Membranen nutzen, unterschieden.

Ein Beispiel für ein intrakorporales Dialyseverfahren ist die Peritonealdialyse, bei der zur Blutreinigung das Peritoneum (Bauchfell) als Membran genutzt wird. Hier wird die Dialysierflüssigkeit durch einen Katheterzugang in die Bauchhöhle des Patienten direkt eingebracht und als Dialysat nach erfolgter Equilibrierung der Stoffkonzentrationen ausgetauscht. Typisch werden so über einen Tag verteilt drei bis sechs Wechsel der Dialyseflüssigkeit mit einem Volumen von jeweils etwa 2,5 bis 4 Litern durchgeführt. Zur Vermeidung von negativen Gesundheitsfolgen für den Patienten muss die Dialysierflüssigkeit vor dem Einbringen in die Bauchhöhle jeweils auf Körpertemperatur gebracht werden.

Ein Beispiel für ein extrakorporales Dialyseverfahren ist die weltweit am meisten angewandte Hämodialyse. Hier wird nach dem Prinzip des Konzentrationsausgleichs kleinmolekularer Substanzen zweier Flüssigkeiten verfahren, die außerhalb des Körpers in einem Dialysator durch eine künstliche semipermeable Dialysemembran getrennt sind. Durch die Dialysemembran getrennt befindet sich auf der einen Seite das Blut mit Nephrotoxinen, Elektrolyten, wie z. B. Kalium und Phosphat, sowie harnpflichtigen Substanzen. Auf der anderen Seite der Dialysemembran befindet sich als Dialysierflüssigkeit eine keimarme, aufbereitete Lösung, die keine Abfallprodukte enthält und einen an den jeweiligen Bedürfnissen des Patienten orientierten Anteil an Elektrolyten aufweist. Die semipermeable Dialysemembran zwischen Blut und Dialyselösung bzw. Dialysierflüssigkeit besitzt Poren, die kleine Moleküle wie Wasser, Elektrolyte und harnpflichtige Substanzen (z. B. Harnstoff, Harnsäure) durchlassen, aber große Moleküle wie Eiweiße und Blutzellen zurückhalten. Typisch sind bei dem Verfahren der Hämodialyse drei bis vier Behandlungen ä etwa 4 h pro Woche. Je Behandlung wird eine erhebliche Menge Dialysierflüssigkeit benötigt, das permanent auf der dem Blut abgewandten Seite der Dialysemembran an der Membran vorbeifließt. Häufig wird dabei die Dialysierflüssigkeit im Gegenstrom zum Blut geführt. Die Dialysierflüssigkeit wird nach einmaliger Verwendung, wenn es mit den harnpflichtigen Substanzen angereichert ist, als Dialysat entsorgt. Allgemein wird aus Gründen einer eindeutigen Zuordnung die zur Dialyse verwendete Flüssigkeit vor der Anreicherung mit harnpflichtigen Stoffen als Dialysierflüssigkeit bezeichnet, während die mit den harnpflichtigen Stoffen angereicherte Flüssigkeit als Dialysat bezeichnet wird. Dabei kann die Menge verbrauchter Dialysierflüssigkeit pro Anwendung 100 1 und mehr betragen. Allein die große Menge herzustellender und verbrauchter Dialysierflüssigkeit macht eine Dialysebehandlung äußerst kostspielig. Zusätzlich zum hohen Ressourcenverbrauch erhöht die zur Aufbereitung der Dialysierflüssigkeit verbrauchte Energie die Kosten erheblich.

Neben der Menge der zugeführten bzw. entnommenen Flüssigkeit aus dem Blut muss auch die Temperatur des Blutes genau kontrolliert werden, um eine schwerwiegende Schädigung des Patienten zu verhindern. Das dem Patienten nach dessen Reinigung wieder zugeführte Blut muss eine von der Körpertemperatur des Patienten abhängige Temperatur aufweisen, um solche Gesundheitsschäden zu vermeiden. Dies wird dadurch erreicht, dass die Dialysierflüssigkeit, und damit auch die Dialysemembran und die diese Membran umgebenden Komponenten auf Körpertemperatur aufgeheizt werden, um ein Abkühlen des Blutes in dem extrakorporalen Kreislauf zu verhindern.

Bevor die Dialysierflüssigkeit mittels eines Dialyseapparats in einem die Filtermembran aufweisenden Dialysator am Blut des Patienten vorbeigeführt wird, wird es also von der Eintrittstemperatur (typ. 10°C) auf eine höhere Temperatur in ungefährer Höhe der Bluttemperatur (typ. 36°C) aufgeheizt. Nachdem das Dialysat durch den Dialysierfilter geströmt ist, ist es gebraucht und gelangt in den Abfluss. Dieses Temperieren der großen Menge von Dialysierflüssigkeit verbraucht eine erhebliche Menge zusätzlicher Energie.

Bei den extrakorporalen Verfahren wie der Hämodialyse (HD) sollte zudem nach jeder Dialysebehandlung der verwendete Apparat, also die Dialysevorrichtung, sterilisiert werden, um eine Kreuz-Infektion zwischen einzelnen Patienten zu verhindern. Zu diesem Zweck wird häufig die so genannte Heißdesinfektion angewendet. Bei dieser wird ein Wasser/ Desinfektionsmittel-Gemisch im Dialysat-Kreislauf der Dialysevorrichtung auf >85°C aufgeheizt und für eine bestimmte Zeit, typisch etwa 15 Minuten, zirkuliert. Nach Ablauf der Zeit sollte das Desinfektionsmittel möglichst schnell ausgespült und die Maschine bzw. die Dialysevorrichtung möglichst schnell wieder auf ca. 35° abgekühlt werden, um die nächste Therapie zu ermöglichen.

Als weitere extrakorporale Dialyseverfahren seien hier nur beispielhaft die Hämofiltration oder die Hämodiafiltration genannt, die zum Teil noch höhere Mengen an Ressourcen und Energie verbrauchen und daher bei ihrer Anwendung ähnliche oder höhere Kosten verursachen.

Beispielsweise zeigt das US Patent 5,861,555 einen herkömmlichen Dialyseapparat, bei dem die Dialysierflüssigkeit mittels zweier hintereinander angebrachter Wärmetauscher temperiert wird, wobei zur Vortemperierung ein Wärmetauscher verwendet wird, der von dem Rücklauf des Dialysats gespeist ist.

Aufgrund des hohen Ressourcenverbrauchs und des hohen Energieverbrauchs bei einer Dialysebehandlung besteht eine erhebliche Notwendigkeit, Dialysevorrichtungen bzw. Verfahren zum Betreiben von Dialysevorrichtungen effizienter zu gestalten.

### Zusammenfassung

Die Aufgabe wird prinzipiell durch eine Dialysevorrichtung mit den Merkmalen des Patentanspruchs 1 gelöst.

Ein Aspekt der vorliegenden Erfindung ist demnach in der Verwendung einer Wärmepumpe zu sehen, um in der erfindungsgemäßen Dialysevorrichtung eine Dialysierflüssigkeit im Zufluss zu einer Dialysemembran zu temperieren. Dies erfolgt vorzugsweise nach dem folgenden Steuerungsprozess:
- Zuleiten der Dialysierflüssigkeit zu der Dialysemembran über den Zufluss,
- Ableiten der verbrauchten Dialysierflüssigkeit von der Dialysemembran über einen Abfluss und
- Temperieren der der Dialysemembran zugeleiteten Dialysierflüssigkeit mit der Wärmepumpe.

Bei einigen Ausführungsbeispielen von Dialysevorrichtungen wird die Wärmepumpe folglich mit einem Zufluss, der dazu dient, eine Dialysierflüssigkeit zu einer Dialysemembran zu leiten, gekoppelt, um Wärmeenergie zwischen einem Wärmereservoir und der Dialysierflüssigkeit im Zufluss auszutauschen. Dies kann zu einer erheblichen Reduktion des Energieverbrauchs bzw. einer erheblichen Erhöhung der Effizienz bei der Temperierung der Dialysierflüssigkeit und somit zu einer Erhöhung der Effizienz der Dialysevorrichtung führen.

Bei einigen Ausführungsbeispielen wird die Wärmepumpe genutzt, um die Dialysierflüssigkeit im Zufluss zu erwärmen. Durch Verwendung einer Wärmepumpe, die verglichen mit anderen Heizmöglichkeiten, wie beispielsweise einer elektrischen Direktheizung, einen erheblich höheren Wirkungsgrad aufweist, kann die Energie, die zum Betrieb einer Dialysevorrichtung erforderlich ist, erheblich reduziert werden. Der höhere Wirkungsgrad bei der Verwendung einer Wärmepumpe rührt daher, dass die von der Wärmepumpe an die Dialysierflüssigkeit abgegebene Wärmeenergie größer ist als die zum Betrieb der Wärmepumpe erforderliche Energie, da die Wärmepumpe die Wärmeenergie vom Wärmereservoir an die Dialysierflüssigkeit transferiert anstatt sie selbst zu erzeugen. Somit ist die Wärmepumpe sowohl anderen Heizungen, die Primärenergieträger direkt in Wärmeenergie umwandeln als auch herkömmlichen Stromheizungen überlegen.

Bei einigen weiteren Ausführungsbeispielen wird ein Umgebungsmedium, wie z. B. die Umgebungsluft, als Wärmereservoir benutzt. Der erzielbare Effizienzgewinn bei der Verwendung 10 einer Wärmepumpe, der sich aus dem Carnot-Prozess ableitet, hängt von der Temperaturdifferenz zwischen den beiden mit der Wärmepumpe gekoppelten Medien ab, vorliegend also von der Temperatur des Wärmereservoirs und der Temperatur der Dialysierflüssigkeit. Dieser auch als Leistungszahl (Coefficient of Performance, COP) bezeichnete Faktor wird umso größer, je höher die Temperatur des Wärmereservoirs relativ zur Temperatur der Dialysierflüssigkeit ist. So kann beispielsweise in südlichen Ländern eine Wärmepumpe im Zufluss einer Dialysevorrichtung auf besonders effiziente Art und Weise die Dialysierflüssigkeit erwärmen, wenn als Wärmereservoir die Umgebungsluft genutzt wird. Gleichzeitig wird bei der Verwendung einer solchen Wärmepumpe die Umgebungsluft gekühlt, so dass diese neben der erforderlichen Funktionalität für die Dialysevorrichtung auch ein angenehmes Klima für den Patienten bewirken kann.

Bei weiteren Ausführungsbeispielen ist die Wärmepumpe zusätzlich mit einem Abfluss der Dialysevorrichtung gekoppelt, der verwendet wird, um das bereits verwendete Dialysat von der Dialysemembran abzuleiten. Hierbei kann das Dialysat im Abfluss als Wärmereservoir verwendet werden.

Bei diesen Ausführungsbeispielen kann in einem ersten Betriebsmodus das vorgeheizte und sich dadurch annähernd auf der Körpertemperatur befindliche, bereits benutzte Dialysat verwendet werden, um als Wärmereservoir bzw. als Wärmequelle für die Wärmepumpe zu dienen. Das heißt, die Energie, die bereits aufgewendet wurde, um das verbrauchte Dialysat zu erwärmen, kann zu großen Teilen wiedergewonnen werden, um die frische Dialysierflüssigkeit im Zufluss aufzuheizen. Dies führt zu einer weiteren erheblichen Reduktion des Energieverbrauchs bei der Dialyse, also insgesamt zu einer Erhöhung der Effizienz beim Betrieb einer Dialysevorrichtung. Dabei ist die Effizienz der Wiedergewinnung von Wärme mittels einer Wärmepumpe aus den oben genannten Gründen erheblich größer als beispielsweise die Effizienz eines herkömmlichen Wärmetauschers.

Wenngleich bei einigen Ausführungsbeispielen das Wärmereservoir eine höhere Temperatur aufweist als die Dialysierflüssigkeit, soll der Begriff Wärmereservoir hier und im Folgenden nicht als in irgendeiner Art von einer Temperatur abhängig verstanden werden. Als Wärmereservoir wird jedwedes Energiereservoir oder Medium verstanden, dem Wärmeenergie entnommen oder zugeführt werden kann.

In einem zweiten Betriebsmodus erlauben es weitere Ausführungsbeispiele von Dialysevorrichtungen, Wärmeenergie von einer Flüssigkeit im Zufluss an das Wärmereservoir zu übertragen, um die Dialysierflüssigkeit oder eine Spülflüssigkeit im Zufluss abzukühlen. In diesem Betriebsmodus kann also beispielsweise das Dialysat oder eine andere Flüssigkeit im Abfluss als Wärmesenke verwendet werden. Dies kann eine Zeit, die eine Dialysevorrichtung nach der Heißdesinfektion benötigt, um auf eine der Körpertemperatur vergleichbare Betriebstemperatur abzukühlen, erheblich verringern, da eine Spülflüssigkeit im Zulauf aktiv gekühlt wird. Dadurch wird sowohl die zur Spülung erforderliche Flüssigkeitsmenge als auch die Spülzeit verringert, was insgesamt zu einer Steigerung der Effizienz beim Betrieb einer Dialysevorrichtung führt.

Bei einigen Ausführungsbeispielen ist in einer Flussrichtung der Dialysierflüssigkeit zwischen der Wärmepumpe und der Dialysemembran ergänzend eine Zusatzheizeinrichtung mit dem Zufluss gekoppelt, um die zufließende, noch unverbrauchte Dialysierflüssigkeit zu erwärmen. Dies kann zu einer Beschleunigung des Aufheizens der Dialysierflüssigkeit zu Beginn des Betriebes der Dialysevorrichtung beitragen, wenn das Dialysat im Abfluss noch nicht die gewünschte Betriebstemperatur erreicht hat. Eine solche Zusatzheizeinrichtung kann beispielsweise eine Stromheizung sein und deutlich kleiner dimensioniert werden, als bei Dialysevorrichtung, die allein über eine herkömmliche Heizeinrichtung verfügen, da die Zusatzheizeinrichtung hier nur unterstützend tätig ist.

Bei einigen Ausführungsbeispielen wird die Zusatzheizeinrichtung dafür verwendet, eine Feinregulierung der Temperatur der Dialysierflüssigkeit im Zufluss vorzunehmen. Dies kann dazu verwendet werden, um sicherzustellen, dass sich die Temperatur der Dialysierflüssigkeit zu jedem Zeitpunkt innerhalb eines erforderlichen Temperaturfensters befindet. Bei einigen Ausführungsbeispielen kann die Zusatzheizeinrichtung auch dazu verwendet werden, um bei einer Reinigung der Dialysevorrichtung eine zur Reinigung verwendete Flüssigkeit auf eine höhere Temperatur aufzuheizen, als die Dialysierflüssigkeit während der Dialyse.

### Figurenkurzbeschreibung

Einige Ausführungsbeispiele der vorliegenden Erfindung werden nachfolgend, bezugnehmend auf die beigefügten Figuren, näher erläutert. Es zeigen:
- Fig. 1: ein Ausführungsbeispiel einer Dialysevorrichtung mit Wärmepumpe;
- Fig. 2: eine Prinzipskizze einer Wärmepumpe;
- Fig. 3: ein weiteres Ausführungsbeispiel einer Dialysevorrichtung, bei der als Wärmereservoir ein Dialysat im Abfluss verwendet wird;
- Fig. 4: ein weiteres Ausführungsbeispiel einer Dialysevorrichtung mit einer Zusatzheizeinrichtung;
- Fig. 5: ein Beispiel für ein Verfahren zum Betreiben einer Dialysevorrichtung; und
- Fig. 6: ein weiteres Beispiel für ein Verfahren zum Betreiben einer Dialysevorrichtung.

### Beschreibung einiger Ausführungsbeispiele

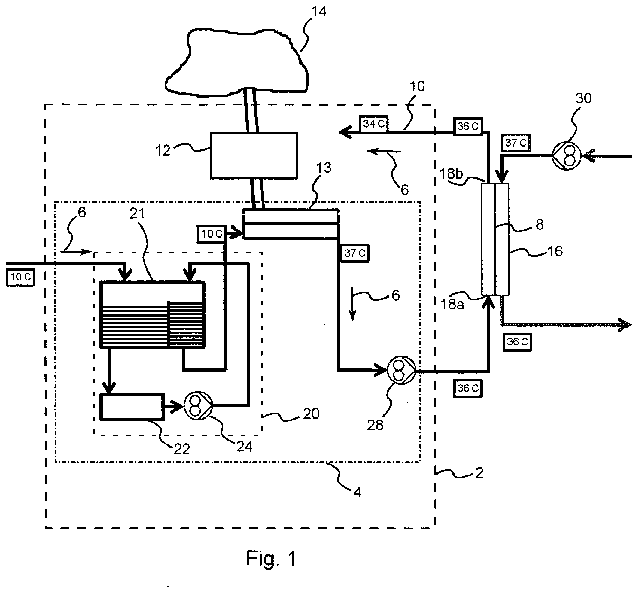

Figur 1 zeigt ein Ausführungsbeispiel einer Dialysevorrichtung 2, die einen Zufluss 4 aufweist, um eine Dialysierflüssigkeit in einer Flussrichtung 6 zu einer Dialysemembran 8 zu leiten. Die Dialysevorrichtung 2 weist ferner einen Abfluss 10 auf, um ein Dialysat von der Dialysemembran 8 abzuleiten. Mit dem Zufluss 4 ist eine Wärmepumpe 12 gekoppelt, die aus gebildet ist, um eine Wärmeenergie zwischen einem Wärmereservoir 14 und der Dialysierflüssigkeit im Zufluss 4 auszutauschen.

Als Abfluss 10 sind vorliegend alle Vorrichtungen bzw. Leitungen zu verstehen, in denen das Dialysat nach dem Vorgang der Dialyse innerhalb der Dialysevorrichtung 2 selbst in der in Figur 1 dargestellten Flussrichtung 6 geführt oder transportiert werden kann. Genauso ist unter dem Zufluss 4 der Dialysevorrichtung 2 jedwede Vorrichtung bzw. Leitung innerhalb der Dialysevorrichtung 2 zu verstehen, die dazu benutzt wird bzw. benutzt werden kann, Dialysierflüssigkeit oder Bestandteile einer Dialysierflüssigkeit zu der Dialysemembran 8 zu leiten. Unter dem Zufluss 4 sollen also insbesondere auch ein Leitungssystem bzw. eine oder mehrere Vorrichtungen verstanden werden, die Dialysierflüssigkeit führen oder verarbeiten. Wie in Figur 1 dargestellt, ist die Dialysemembran 8 bzw. ein die Dialysemembran 8 enthaltender Dialysator 16 gemäß manchen Ausführungsbeispielen nicht Bestandteil der Dialysevorrichtung 2 selbst. Vielmehr kann der Dialysator 16 zusammen mit der Dialysemembran 8 nach jeder Behandlung ausgetauscht werden, so dass es bei der darauf folgenden Behandlung eines anderen Patienten zu keiner Kreuzinfektionen kommen kann. Der Dialysator 16 weist dazu Anschlüsse 18a und 18b auf, um den Zufluss 4 und den Abfluss 10 der Dialysevorrichtung 2 mit dem Dialysator 16 zu koppeln, so dass die Dialysierflüssigkeit auf einer Seite (in Fig. 1 links) der Dialysemembran 8 durch das Dialysatorgehäuse 16 geleitet wird.

Um das Blut des Patienten auf der anderen Seite (in Fig. 1 rechts dargestellt) der Dialysemembran 8 vorbeizuführen, ist ein weiterer, hier nur der Vollständigkeit halber erwähnter, Blutzirkulationskreislauf erforderlich, der insbesondere bei veno- venösem Zugang beim Patienten eine Blutförderpumpe 30 umfassen kann, wie sie in Fig. 1 dargestellt ist.

Die Wärmepumpe 12 ist, wie in Figur 1 dargestellt, derart mit dem Zufluss bzw. Zulauf 4 gekoppelt, dass mittels der Wärmepumpe 12 Wärmeenergie zwischen einem Wärmereservoir 14 und der Dialysierflüssigkeit im Zufluss 4 ausgetauscht werden kann. Das für einen solchen Austausch von Wärmeenergie mittels einer Wärmepumpe 12 zusätzlich notwendige Medium, welches das Wärmereservoir bildet, ist in Figur 1 nur schematisch dargestellt. Wenngleich bei dem in Figur 1 gezeigten Ausführungsbeispiel, ebenso wie in den nachfolgend beschriebenen Ausführungsbeispielen, die Wärmepumpe 12 mittels eines Wärmetauschers 13 mit der Dialysierflüssigkeit im Zufluss 4 gekoppelt ist, kann sie bei weiteren Ausführungsbeispielen auch auf andere Art und Weise mit dem Zufluss 4 gekoppelt sein, um Wärmeenergie mit dem Zufluss 4 bzw. der Dialysierflüssigkeit oder einer anderen Flüssigkeit im Zufluss 4 auszutauschen. Dies kann beispielsweise mittels Strahlung der Fall sein.

Als weitere optionale Komponenten zeigt die in Figur 1 dargestellte Dialysevorrichtung 2 eine Entgasungsvorrichtung 20, die aus einem Flüssigkeitsreservoir 21, einer Entgasungseinrichtung 22 und einer Kreislaufpumpe 24 besteht. Der in Figur 1 schematisch dargestellte Kreislauf innerhalb der Entgasungsvorrichtung 20 dient dazu, die Dialysierflüssigkeit zu entgasen, wozu diese zunächst von einem ersten Teilreservoir innerhalb des Flüssigkeitsreservoirs 21 über die Entgasungseinrichtung 22 in ein zweites Teilreservoir geleitet wird. Zu diesem Zweck ist bei dem in Figur 1 dargestellten Ausführungsbeispielen eine Förderpumpe 24 vorgesehen, die diesen Teilkreislauf für die Entgasung antreibt.

Die entgaste Dialysierflüssigkeit wird nach der Entgasungsvorrichtung 20, die in Flussrichtung 6 vor der Wärmepumpe 12 angeordnet ist, an derjenigen Stelle vorbeitransportiert, an der die Wärmepumpe 12 mit dem Zufluss 4 gekoppelt ist. Die Wärmepumpe 12 dient dazu, dass Dialysat im Zufluss 4 während der Dialyse zu erwärmen, so dass dieses, wenn es die Dialysemembran 8 erreicht, zumindest annähernd Körpertemperatur aufweist. Um den Kreislauf der Dialysierflüssigkeit und des Dialysats aufrechtzuerhalten, weist das in Figur 1 dargestellte Ausführungsbeispiel ferner eine weitere optionale Förderpumpe 28 auf, die die Dialysierflüssigkeit nach deren Erwärmung mittels der Wärmepumpe 12 in Richtung der Dialysemembran 8 transportiert.

In Figur 1 sind exemplarisch typische Temperaturen der Dialysierflüssigkeit und des Dialysats vor bzw. nach der Erwärmung dargestellt. Beispielsweise kann die angelieferte Flüssigkeit bzw Dialysierflüssigkeit mit einer Temperatur von etwa 10°C angeliefert werden. Vor dem Durchströmen des externen Dialysators 16 wird bei dem in Figur 1 dargestellten Ausführungsbeispiel die Temperatur auf etwa 37°C erhöht. Dies geschieht mittels der Wärmepumpe 12, die eine dazu nötige Wärmeenergie dem Wärmereservoir 14 entnimmt.

Es versteht sich von selbst, dass hier, wie auch in den nachfolgend diskutierten Ausführungsbeispielen, die exakte Position der Kopplung der einzelnen Komponenten mit dem Zufluss 4 bzw. deren Position innerhalb des Zuflusses 4 beliebig miteinander ausgetauscht werden können. Beispielsweise kann die Entgasungsvorrichtung 20 in Flussrichtung 6 auch zwischen einer Koppelstelle der Wärmepumpe 12 und der Dialysemembran 8 bzw. zwischen der Wärmepumpe 12 und der Förderpumpe 28 angeordnet sein. Selbstverständlich sind auch die angegebenen Temperaturen nur als Beispiele zu verstehen, es sind beliebige andere Temperaturen möglich. Insbesondere in wärmeren Ländern oder zu wärmeren Jahreszeiten kann die Temperatur des eingespeisten Wassers bzw. der Dialysierflüssigkeit am Anfang des Zuflusses 4 auch deutlich höher liegen als 10°C, sodass als Wärmereservoir 14 beispielsweise die Umgebungsluft verwendet werden kann.

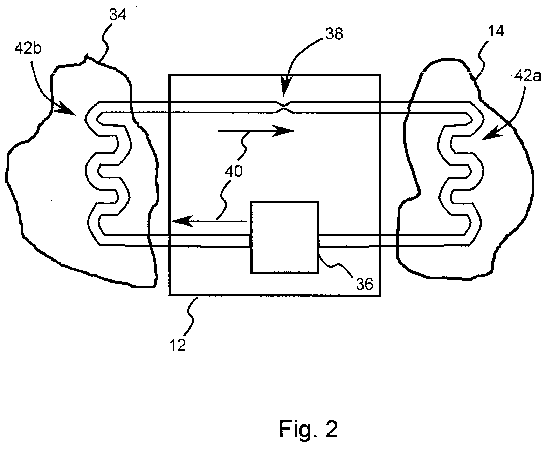

Anhand von Figur 2 wird zum besseren Verständnis des Wärmetransports zwischen dem Wärmereservoir 14 und dem Zufluss 4 kurz die Funktionsweise der Wärmepumpe 12 erläutert.

Die Wärmepumpe 12 ist eine Maschine, die unter Zufuhr von technischer Arbeit Wärme von einer Wärmequelle bzw. einem Wärmereservoir 14 zu einem zu erwärmenden Medium 34 pumpt. Entwickelt wurde die Wärmepumpe, um Wärmeenergie von einem niedrigeren zu einem höheren Temperaturniveau zu pumpen, sodass die Wärmeenergie, die in dem niedriger temperierten Medium enthalten ist, zum zusätzlichen Heizen eines Mediums auf höherer Temperatur genutzt werden kann, was mittels normaler Wärmetauscher nicht möglich wäre.

Bei einer Wärmepumpenheizung wird die auf einem hohen Temperaturniveau anfallende Verflüssigungswärme eines in dem Wärmepumpenkreislauf verwendeten Arbeitsmediums zum Heizen genutzt. Dagegen wird bei einer Kältemaschine die Abkühlung eines Kältemittels beim Entspannen und Verdampfen genutzt, um ein Fluid abzukühlen. Wärmepumpen und Kältemaschinen unterscheiden sich lediglich hinsichtlich der genutzten Energie, der entweder zum Abkühlen oder zum Aufwärmen genutzten Wärmeleistung. Der grundlegende Prozess, der in Figur 2 dargestellt ist, ist jedoch derselbe.

Die in Figur 2 dargestellte Wärmepumpe 12 weist einen Verdichter bzw. Verflüssiger 36 und einen Verdampfer bzw. ein Expansionsventil 38 auf. Ein Arbeitsmedium, oft auch als Kältemittel bezeichnet, zirkuliert in einer Zirkulationsrichtung 40 innerhalb eines von dem Verdichter 36 angetriebenen Kreislaufs. Der Verdichter 36 kann beispielsweise elektrisch oder durch einen Verbrennungsmotor angetrieben werden und bewirkt die Zirkulation des Arbeitsmediums innerhalb des Kreislaufs. Der Verdichter 36 komprimiert das Arbeitsmedium bzw. das Kältemittel auf einen höheren Druck wobei sich das üblicherweise vor dem Verdichter 36 gasförmige Arbeitsmedium erwärmt und anschließend verflüssigt. Die beim Verflüssigen des Kältemittels freigesetzte Energie wird in einem Wärmeübertrager 42b auf das zu erwärmende Medium 34 übertragen. Als Wärmeübertrager 42b kann beispielsweise ein Wärmetauscher genutzt werden. Alternativ kann die Wärme auch durch andere Mechanismen, beispielsweise durch Strahlung auf das zu erwärmende Medium übertragen werden. Allgemein gesprochen ist die Wärmepumpe 12 bzw. das Arbeitsmedium bei den Ausführungsbeispielen der Erfindung derart mit dem zu erwärmenden Medium 34 gekoppelt, dass ein Austausch von Wärmeenergie möglich ist.

Nach dem Übertragen der Wärmeenergie wird das Arbeitsmedium bzw. das Kältemittel an einem Dekomprimierer bzw. an einem Expansionsventil 38 entspannt, wobei es sich abkühlt. Das kalte Kältemittel wird dann einem Verdampfer 42a (Wasser-Wärmetauscher, Erdwärmesonden, Luftverdampfer) zugeführt und geht durch Aufnahme von Umgebungswärme (Anergie) aus der Wärmequelle bzw. aus dem Wärmereservoir 14 in den gasförmigen Zustand über. Bezüglich der Kopplung des Verdampfers mit dem Wärmereservoir 14 gilt das oben zu der Kopplung mit dem zu erwärmende Medium 34 gesagte.

Das Verhältnis von nutzbarer Wärmeleistung zu zugeführter elektrischer Leistung wird als Leistungszahl bzw. in der Fachliteratur als COP ("Coefficient of Performance") der Wärmepumpe bezeichnet. Die Leistungszahl hat einen theoretischen Maximalwert, der von den Temperaturen des Wärmereservoirs 14 und des zu erwärmenden Mediums 34 abhängt und aus dem Carnot-Prozess (benannt nach Nicolas Leonard Sadi Carnot) abgeleitet werden kann.

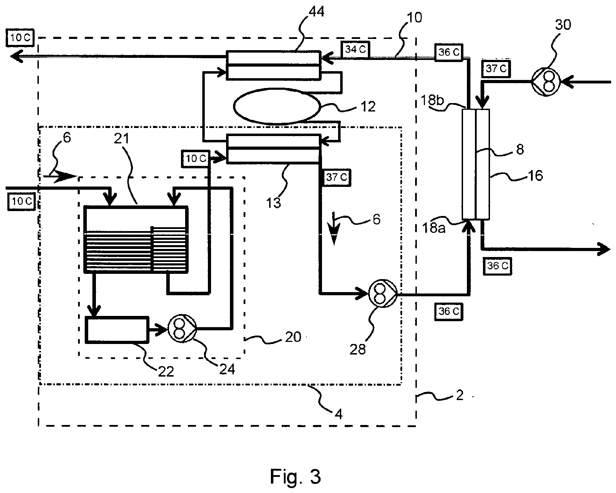

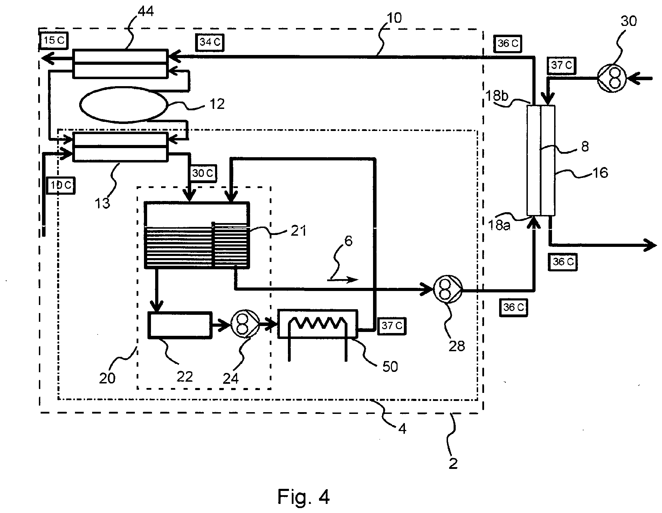

Bei typischen Temperaturen hat der COP einen Wert von 8 oder darüber. In der Praxis derzeit erzielbare Werte liegen zwischen 4 und 6. Dies bedeutet, dass das 4- bis 6- fache derjenigen Energie, die zum Antrieb der Wärmepumpe 12 benötigt wird, an das zu erwärmende Medium 34übertragen wird.BeimEinsatz derWärmepumpe 12 in denDialysevorrichtungen der Figuren 3 und 4, wo entgegen der üblichen Betriebsart der Wärmepumpe 12 Wärmeenergie von einem Reservoir höherer Temperatur zu einem Medium niederer Temperatur übertragen wird, liegen die praktisch erzielten Werte aufgrund dieser Tatsache noch darüber.

Nimmt man beispielsweise an, die Dialysierflüssigkeit muss im Zufluss 4 von 10°C auf etwa 36°C erhitzt werden, und werden für eine Behandlung ca. 120 1 benötigt, führt dies zu einem Wärmeenergiebedarf von ca. 3,6 kWh, sofern man idealisierend annimmt, die Wärmeenergie könnte bei der Aufheizung verlustfrei aus einem anderen Energieträger erzeugt werden, was in der Realität natürlich nicht der Fall ist.

Selbst bei der Verwendung eines Wärmetauschers zwischen dem Dialysat im Abfluss 10 und der Dialysierflüssigkeit im Zufluss 4 kann nur ein kleiner Teil der eingesetzten Energie wiederverwendet werden. Wirkungsgrade für sehr gute Wärmetauscher liegen typischerweise bei weniger als 60% - 70%, wobei ein Wärmeenergieübertrag nur stattfinden kann, solange das Dialysat im Abfluss 10 noch wärmer ist als die Dialysierflüssigkeit im Zufluss 4. Dies ist bei Verwendung einer Wärmepumpe kein limitierender Faktor. Selbst wenn man einen sehr guten Wärmetauscher annähme, ergibt sich für eine herkömmliche Dialysevorrichtung immer noch ein Mindestenergiebedarf von ca. 1,80 kWh zur Heizung der Dialysierflüssigkeit für eine typische Behandlung.

Dieser Energiebedarf kann bei Verwendung einer Wärmepumpe erheblich reduziert werden. Unter der Annahme, man würde die gesamte zur anfänglichen Erwärmung der Dialysierflüssigkeit im Zufluss 4 verwendete Energie von dem Dialysat im Abfluss 10 mittels einer Wärmepumpe an die Dialysierflüssigkeit im Zufluss 4 übertragen, sodass das Dialysat im Abfluss 10 in etwa dieselbe Temperatur hätte wie die Dialysierflüssigkeit im Zufluss 4 vor dem Erwärmen, ergibt sich selbst bei einem minimal zu erwartenden COP von 4 bereits eine Energieersparnis von 50%, da nur ein Viertel der übertragenen Wärmeenergie als Energie zum Antrieb der Wärmepumpe verwendet werden muss. Im idealisierten Beispiel kann also der Energiebedarf auf unter 0,9 kWh reduziert werden. Unter Berücksichtigung der tatsächlich auftretenden zusätzlichen Verluste in herkömmlichen Dialysevorrichtungen, kann die erzielbare Effizienzsteigerung erheblich höher sein.

Figur 3 zeigt ein weiteres Ausführungsbeispiel einer Dialysevorrichtung 2, das einige Komponenten mit dem Ausführungsbeispiel, das anhand von Figur 1 dargestellt wurde, gemeinsam hat. Daher wird im Folgenden nur auf die Unterschiede zu dem in Figur 1 gezeigten Ausführungsbeispiel näher eingegangen. Generell sind funktionsähnliche oder -gleiche Komponenten bei den hierin beschriebenen Ausführungsbeispielen mit denselben Bezugszeichen versehen. Die anhand der verschiedenen Ausführungsbeispiele beschriebenen Komponenten bzw. deren Funktionalität kann darüber hinaus innerhalb der einzelnen Ausführungsbeispiele beliebig ausgetauscht werden.

Bei dem in Figur 3 gezeigten Ausführungsbeispiel verwendet die Wärmepumpe 12 als Wärmereservoir das Dialysat im Abfluss 10. Zu diesem Zweck ist die Wärmepumpe 12, beispielsweise mittels eines Wärmetauschers 44 mit dem Dialysat im Abfluss 10 gekoppelt, um das abfließende Dialysat als Wärmereservoir verwenden zu können. Während der Dialyse (des ersten Betriebsmoduses der Wärmepumpe in Figur 3) zirkuliert der in der Wärmepumpe 12 enthaltene Kompressor bzw. Verdichter 36 das Arbeitsmedium der Wärmepumpe 12 in der in Figur 3 angedeuteten Richtung, um Wärmeenergie von dem Dialysat im Abfluss 10 zu der Dialysierflüssigkeit im Zufluss 4 zu übertragen. Da der Prozess, der in der Wärmepumpe 12 zum Übertragen der Wärmeenergie führt, reversibel ist, kann die Wärmepumpe 12 in einem zweiten Betriebsmodus, in dem das Arbeitsmittel in der anderen Richtung zirkuliert, auch Wärmeenergie von der Dialysierflüssigkeit bzw. einer Flüssigkeit im Zufluss 4 zu dem Dialysat bzw. der Flüssigkeit im Abfluss 10 übertragen.

Dieser zweite Betriebsmodus kann während oder nach der Reinigung einer Dialysevorrichtung von großem Vorteil sein. Während der Ausspülphase am Ende der Heißdesinfektion soll die Dialysevorrichtung möglichst schnell wieder eine Temperatur <40 °C erreichen, um mit der nächsten Behandlung beginnen zu können. Die Heißdesinfektion wird mit >85°C heißem Wasser, dem Desinfektionsmittel zugesetzt ist, durchgeführt. Verwendete man zur Energierückgewinnung einen Wärmetauscher, würde beim Ausspülen der Dialysevorrichtung nach der Desinfektion das zulaufende Frischwasser unerwünscht durch den Wärmetauscher aufgeheizt, was die Ausspülphase verlängert. Diese Effizienzverringerung wird bei Verwendung einer Wärmepumpe vermieden. Bei der Implementierung des oben beschriebenen zweiten Betriebsmodus kann eine Flüssigkeit im Zufluss 4 sogar aktiv gekühlt werden, was zu einer weiteren Effizienzsteigerung führt.

Unabhängig davon hat die Verwendung einer Wärmepumpe auch während des Dialysebetriebs den Vorteil, dass die Temperatur des aus dem Abfluss 10 austretenden Dialysats geringer ist als dies herkömmlicherweise der Fall ist. Dies ist in Kliniken von großem Vorteil, da dadurch ein Keimwachstum im Abfluss bzw. Ablauf weniger begünstigt wird, als dies der Fall ist, wenn das ablaufende Dialysat eine Temperatur von 20° oder mehr aufweist.

Figur 4 zeigt ein weiteres Ausführungsbeispiel einer Dialysevorrichtung, bei der zusätzlich eine Zusatzheizeinrichtung 50 mit dem Zufluss 4 gekoppelt ist, um die Dialysierflüssigkeit im Zufluss 4 zu erwärmen. Bei dem in Figur 4 gezeigten Ausführungsbeispiel ist die Zusatzheizeinrichtung 50 in der Flussrichtung 6 zwischen der Wärmepumpe 12 und der Dialysemembran 8 angeordnet, um die Dialysierflüssigkeit im Zufluss 4 zusätzlich und unabhängig von der Wärmepumpe 12 zu erwärmen. Dies kann bei der Inbetriebnahme der Dialysevorrichtung dazu verwendet werden, die Zeit, die dazu benötigt wird, die Dialysierflüssigkeit auf die erforderliche Temperatur aufzuheizen, zu verringern, wenn zu Beginn das Dialysat im Abfluss 10 noch nicht die im stationären Betrieb erzielte Temperatur aufweist. Ferner kann bei einigen Ausführungsbeispielen durch die Verwendung beispielsweise einer Stromheizung oder einer schnellregelnden Heizung als Zusatzheizeinrichtung 50 sichergestellt werden, dass zu jedem Zeitpunkt die Temperatur der Dialysierflüssigkeit in einem erforderlichen Temperaturfenster liegt.

Es versteht sich von selbst, dass die Zusatzheizeinrichtung 50 an beliebiger anderer Stelle mit dem Zufluss 4 gekoppelt sein kann, um die Dialysierflüssigkeit im Zufluss 4 zu erhitzen. Beispielsweise kann die Zusatzheizeinrichtung 50 auch zwischen der Förderpumpe 28 und der Dialysemembran 8 angeordnet sein. Insbesondere kann die Zusatzheizeinrichtung 50 auch außerhalb des Kreislaufs der Entgasungsvorrichtung 20 angeordnet sein.

Mit anderen Worten ausgedrückt, zeigt Figur 4 ein Ausführungsbeispiel, bei dem eine Wärmepumpe 12 dazu genutzt wird, um die hauptsächliche Heizleistung der Dialysevorrichtung zu erbringen. Eine kleine Heizung ist vorhanden, um damit eine genaue Regelung der Temperatur zu erreichen. Die Menge an zulaufendem Wasser bzw. Dialysierflüssigkeit und ablaufenden Dialysat ist weitgehend gleich. Das bedeutet, wenn es gelingt, die Wärme aus dem Abwasser über die Wärmepumpe 12 dem Zulauf bzw. Zufluss 4 zur Verfügung zu stellen, dann muss als Differenzenergie nur das zugeführt werden, was an Verlusten durch Konvektion oder Strahlung entsteht. Die Wärmepumpe 12 entzieht in dem Verdampfer auf der Ablaufseite dem ablaufenden und verbrauchten Dialysat soweit möglich die Energie. Die Wärmepumpe 12 verdichtet das Kühlmittel. Auf der anderen Seite des Wärmekreislaufs der Wärmepumpe 12 sitzt ein Verflüssiger im Wasserzulauf bzw. Zufluss. Dort wird die Energie durch das Verflüssigen an das zulaufende Wasser bzw. die zulaufende Dialysierflüssigkeit abgegeben. Die Wärmepumpe 12 erhitzt also die zulaufende Dialysierflüssigkeit mit der aus dem Ablauf entzogenen Energie. Die Temperatur des ablaufenden Dialysats kann dabei sogar unter der des zulaufenden Wassers liegen.

Die Heizung bzw. Zusatzheizeinrichtung 50 kann die Aufheizphase bei Beginn der Behandlung beschleunigen, wenn das Wasser bzw. das Dialysat im Ablauf bzw. Abfluss 10 noch keine Betriebstemperatur aufweist. Die Heizung kann weiterhin dazu dienen, die Temperaturregelgenauigkeit zu erhöhen. Dies ist jedoch nur eine mögliche Ausführungsform. Es könnte auch ein Mischventil oder Ähnliches für die Temperaturregelung verwendet werden.

Zusammenfassend kann festgestellt werden dass einige Ausführungsbeispiele der Erfindung jeweils einen oder mehrere der folgenden Vorteile bieten:
- Die Energieeffizienz der Maschine wird erhöht, da dem ab laufenden Dialysat mehr als die beim Aufheizen zugeführte Energie entzogen werden kann.
- Ein Abkühlen nach der Desinfektion wird ermöglicht. Die Wärmepumpe 12 kann so ausgeführt werden, dass sie während der Abkühlphase im Umkehrbetrieb arbeitet oder abgeschaltet ist, was beides das Abkühlen beschleunigt.
- Das ablaufende Dialysat kann kälter als bei herkömmlichen Verfahren sein. Dies kann günstig sein, um Keimwachstum im Abflusssystem zu verringern.

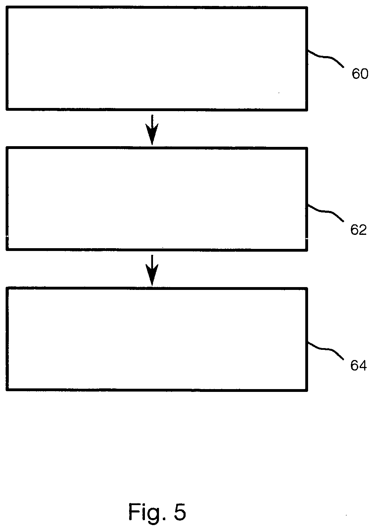

Figur 5 zeigt ein Beispiel für ein Verfahren zu effizienten Betrieb einer Dialysevorrichtung.

Beim Zuleiten 60 wird einer Dialysemembran 8 eine Dialysierflüssigkeit über einen Zufluss 4 zugeleitet. Beim Ableiten 62 wird ein Dialysat von der Dialysemembran 8 über einen Abfluss 10 abgeleitet. Während des Temperierens 64 wird die der Dialysemembran zugeleiteten Dialysierflüssigkeit mit einer Wärmepumpe 12 temperiert.

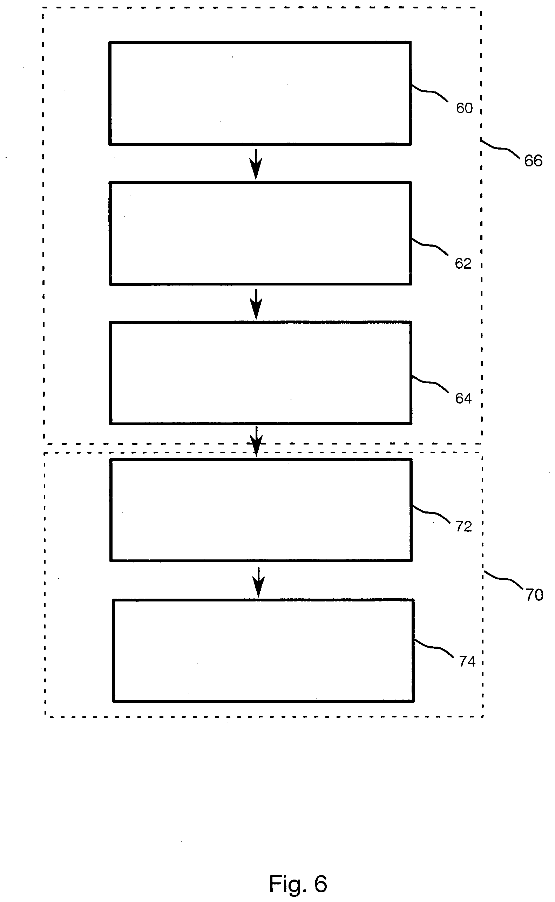

Figur 6 zeigt ein weiteres Beispiel für ein Verfahren zum effizienten Betrieb einer Dialysevorrichtung, das in einem ersten Betriebsmodus 66 die Schritte des in Figur 5 gezeigten Verfahrens umfasst, wobei die der Dialysemembran 8 zugeleitete Dialysierflüssigkeit mit der Wärmepumpe 12 erwärmt wird.

In einem optionalen zweiten Reinigungsmodus 70 wird während einer Reinigung 72 der Zufluss 4 und der Abfluss 10 mit einer den Zufluss 4 und den Abfluss 10 durchströmenden Reinigungsflüssigkeit gereinigt.

Während eines Spülens 74 der Dialysevorrichtung wird eine Spülflüssigkeit oder die Dialysierflüssigkeit im Zufluss 4 mittels der in einem zweiten Betriebsmodus operierenden Wärmepumpe 12 gekühlt.

## Patentansprüche

1. Dialysevorrichtung (2), umfassend folgende Merkmale:
einen Zufluss (4), um eine Dialysierflüssigkeit in einer Flussrichtung (6) zu einer Dialysemembran (8) zu leiten;
einen Abfluss (10), um ein Dialysat von der Dialysemembran (8) abzuleiten,
**gekennzeichnet durch** eine mit dem Zufluss (4) gekoppelte Wärmepumpe (12), die ausgebildet ist, um Wärmeenergie zwischen einem Wärmereservoir (14) und der Dialysierflüssigkeit im Zufluss (4) auszutauschen, wobei die Dialysevorrichtung (2) ferner eine mit dem Zufluss (4) gekoppelte Entgasungsvorrichtung (20) aufweist, die ausgebildet ist, um in der Dialysierflüssigkeit enthaltene Gase zu entfernen.

2. Dialysevorrichtung (2) gemäß Anspruch 1, bei der die Wärmepumpe (12) ausgebildet ist, um eine Umgebungsluft als Wärmereservoir (14) zu nutzen.

3. Dialysevorrichtung (2) gemäß Anspruch 1, bei der die Wärmepumpe (12) ferner mit dem Abfluss (10) gekoppelt ist, wobei die Wärmepumpe (12) ausgebildet ist, um das Dialysat im Abfluss (10) als Wärmereservoir (14) zu verwenden.

4. Dialysevorrichtung (2) gemäß einem der vorhergehenden Ansprüche, bei der die Wärmepumpe (12) aus gebildet ist, in einem ersten Betriebsmodus Wärmeenergie von dem Wärmereservoir (14) an die Dialysierflüssigkeit im Zufluss (4) zu übertragen, um die Dialysierflüssigkeit zu erwärmen.

5. Dialysevorrichtung (2) gemäß einem der vorhergehenden Ansprüche, bei der die Wärmepumpe (12) ausgebildet ist, in einem zweiten Betriebsmodus Wärmeenergie von der Dialysierflüssigkeit im Zufluss (4) an das Wärmereservoir (14) zu übertragen, um die Dialysierflüssigkeit abzukühlen.

6. Dialysevorrichtung (2) gemäß einem der vorhergehenden Ansprüche, die im Zufluss (4) ferner eine in der Flussrichtung (6) zwischen der Wärmepumpe (12) und der Dialysemembran (8) mit dem Zufluss (4) gekoppelte Zusatzheizeinrichtung (50) aufweist, die ausgebildet ist, um die Dialysierflüssigkeit im Zufluss (4) zu erwärmen.

7. Dialysevorrichtung (2) gemäß Anspruch 5, bei der die Zusatzheizeinrichtung (50) eine elektrisch betriebene Heizung ist.

8. Dialysevorrichtung (2) gemäß einem der vorhergehenden Ansprüche, die ferner eine in der Flussrichtung (6) zwischen der Wärmepumpe (12) und der Dialysemembran (8) angeordnete Förderpumpe (28) aufweist.

9. Dialysevorrichtung (2) gemäß Anspruch 1, bei der die Entgasungsvorrichtung (20) in der Flussrichtung (6) zwischen der Wärmepumpe (12) und der Dialysemembran (8) oder in der Flussrichtung (6) vor der Wärmepumpe (12) mit dem Zufluss (4) gekoppelt ist.

10. Dialysevorrichtung (2) gemäß einem der vorhergehenden Ansprüche, die ferner einen Dialysator (16) mit einer Dialysemembran (8) aufweist, wobei der Zufluss (4) und der Abfluss (10) mit dem Dialysator (16) gekoppelt sind und wobei sich an dem Dialysator (16) ferner Anschlüsse für einen Zulauf und einen Ablauf eines mittels der Dialysevorrichtung (2) zu reinigenden Blutes befinden.

11. Dialysevorrichtung (2), nach einem der vorstehenden Ansprüche, **dadurch gekennzeichnet, dass** Dialysierflüssigkeit zu der Dialysemembran (8) über den Zufluss (4) zugeleitet wird, dass Dialysat von der Dialysemembran (8) über den Abfluss (10) abgeleitet wird; und dass die der Dialysemembran (8) zugeleitete Dialysierflüssigkeit mit der Wärmepumpe (12), temperiert wird.

12. Dialysevorrichtung (2) nach einem der vorstehenden Ansprüche, **dadurch gekennzeichnet, dass** in einem ersten Betriebsmodus die der Dialysemembran (8) zugeleitete Dialysierflüssigkeit mit der Wärmepumpe (12) erwärmt wird.

13. Dialysevorrichtung (2) gemäß Anspruch 12, **dadurch gekennzeichnet, dass** der Zufluss (4) und der Abfluss mit einer den Zufluss (4) und den Abfluss durchströmenden Reinigungsflüssigkeit-gereinigt wird und dann die Dialysevorrichtung (2) gespült wird, wobei während des Spülens in einem zweiten Betriebsmodus eine Spülflüssigkeit oder die Dialysierflüssigkeit im Zufluss (4) mittels der Wärmepumpe (12) gekühlt wird.

## Claims

1. A dialysis unit (2) comprising the following features:
an inlet means (4) for guiding a dialysis fluid in a direction of flow (6) to a dialyzer membrane (8);
an outlet means (10) for discharging a dialysate from the dialyzer membrane (8);
**characterized by** a heat pump (12) coupled to the inlet means (4), which is configured to exchange thermal energy between a heat reservoir (14) and the dialysis fluid in the inlet (4), the dialysis unit (2) further comprising a degassing device (20) coupled to the inlet means (4), which is configured to remove gases contained in the dialysis fluid.

2. The dialysis unit (2) according to claim 1 in which the heat pump (12) is configured to utilize ambient air as heat reservoir (14).

3. The dialysis unit (2) according to claim 1, in which the heat pump (12) is further coupled to the outlet (10), wherein the heat pump (12) is configured to use the dialysate in the outlet means (10) as heat reservoir (14).

4. The dialysis unit (2) according to any one of the preceding claims, in which the heat pump (12) is configured to transfer, in a first operating mode, thermal energy from the heat reservoir (14) to the dialysis fluid in the inlet means (4) so as to heat the dialysis fluid.

5. The dialysis unit (2) according to any one of the preceding claims, in which the heat pump (12) is configured to transfer, in a second operating mode, thermal energy from the dialysis fluid in the inlet means (4) to the heat reservoir (14) so as to cool the dialysis fluid.

6. The dialysis unit (2) according to any one of the preceding claims, further comprising in the inlet means (4) an extra heating device (50) coupled to the inlet means (4) in the direction of flow (6) between the heat pump (12) and the dialyzer membrane (8), the extra heating device (50) being configured to heat the dialysis fluid in the inlet means (4).

7. The dialysis unit (2) according to claim 5, in which the extra heating device (50) is an electrically operated heating.

8. The dialysis unit (2) according to any one of the preceding claims, further comprising a delivery pump (28) arranged in the direction of flow (6) between the heat pump (12) and the dialyzer membrane (8).

9. The dialysis unit (2) according to claim 1, in which the degassing device (20) is coupled to the inlet means (4) in the direction of flow (6) between the heat pump (12) and the dialyzer membrane (8), or in the direction of flow (6) ahead of the heat pump (12).

10. The dialysis unit (2) according to any one of the preceding claims, further comprising a dialyzer (16) having a dialyzer membrane (8), wherein the inlet means (4) and the outlet means (10) are coupled to the dialyzer (16) and wherein at the dialyzer (16) furthermore ports for an inflow and an outflow of blood to be purified by means of the dialysis unit (2) are arranged.

11. The dialysis unit (2) according to any one of the preceding claims, **characterized in that** a dialysis fluid is supplied to the dialyzer membrane (8) via the inlet means (4); that dialysate from the dialyzer membrane (8) is discharged via the outlet means (10); and that the dialysis fluid supplied to the dialyzer membrane (8) is temperature-controlled by the heat pump (12).

12. The dialysis unit (2) according to any one of the preceding claims, **characterized in that** in a first operating mode, the dialysis fluid supplied to the dialyzer membrane (8) is heated by the heat pump (12).

13. The dialysis unit (2) according to claim 12, **characterized in that** the inlet means (4) and the outlet means is cleaned with a cleaning fluid flowing through the inlet means (4) and the outlet means, and the dialysis unit (2) is then rinsed, wherein during rinsing in a second operating mode, a rinsing fluid or the dialysis fluid is cooled in the inlet means (4) by means of the heat pump (12).

## Revendications

1. Dispositif de dialyse (2) comprenant les caractéristiques suivantes :
un circuit d'acheminement (4) pour conduire un liquide de dialyse dans une direction d'écoulement (6) à une membrane de dialyse (8) ;
un circuit d'évacuation (10) pour évacuer un dialysat de la membrane de dialyse (8),
**caractérisé par** une pompe à chaleur (12) couplée au circuit d'acheminement (4) qui est conçue pour échanger de l'énergie thermique entre un réservoir de chaleur (14) et le liquide de dialyse dans le circuit d'acheminement (4), dans lequel le dispositif de dialyse (2) présente de plus un dispositif de dégazage (20) couplé au circuit d'acheminement (4) qui est conçu pour éliminer les gaz contenus dans le liquide de dialyse.

2. Dispositif de dialyse (2) selon la revendication 1, pour lequel la pompe à chaleur (12) est conçue pour utiliser un air ambiant comme réservoir de chaleur (14).

3. Dispositif de dialyse (2) selon la revendication 1, pour lequel la pompe à chaleur (12) est en outre couplée au circuit d'évacuation (10), dans lequel la pompe à chaleur (12) est conçue pour utiliser le dialysat dans le circuit d'évacuation (10) comme réservoir de chaleur (14).

4. Dispositif de dialyse (2) selon l'une des revendications précédentes, dans lequel la pompe à chaleur (12) est conçue pour transmettre dans un premier mode de fonctionnement de l'énergie thermique du réservoir de chaleur (14) au liquide de dialyse dans le circuit d'acheminement (4) pour chauffer le liquide de dialyse.

5. Dispositif de dialyse (2) selon l'une des revendications précédentes, dans lequel la pompe à chaleur (12) est conçue pour transmettre dans un deuxième mode de fonctionnement, de l'énergie thermique du liquide de dialyse dans le circuit d'acheminement (4) au réservoir de chaleur (14) pour refroidir le liquide de dialyse.

6. Dispositif de dialyse (2) selon l'une des revendications précédentes, qui présente en outre dans le circuit d'acheminement (4) un système de chauffage d'addition (50) couplé au circuit d'acheminement (4) dans la direction d'écoulement (6) entre la pompe à chaleur (12) et la membrane de dialyse (8), qui est conçu pour réchauffer le liquide de dialyse dans le circuit d'acheminement (4).

7. Dispositif de dialyse (2) selon la revendication 5, dans lequel le système de chauffage d'addition (50) est un chauffage actionné électriquement.

8. Dispositif de dialyse (2) selon l'une des revendications précédentes, qui présente en outre une pompe d'alimentation (28) disposée dans la direction d'écoulement (6) entre la pompe à chaleur (12) et la membrane de dialyse (8).

9. Dispositif de dialyse (2) selon la revendication 1, dans lequel le dispositif de dégazage (20) est couplé dans la direction d'écoulement (6) entre la pompe à chaleur (12) et la membrane de dialyse (8) ou dans la direction d'écoulement (6) avant la pompe à chaleur (12), au circuit d'acheminement (4).

10. Dispositif de dialyse (2) selon l'une des revendications précédentes, qui présente en outre un dialyseur (16) comprenant une membrane de dialyse (8), dans lequel le circuit d'acheminement (4) et le circuit d'évacuation (10) sont couplés au dialyseur (16) et dans lequel se trouvent en outre sur le dialyseur (16), des raccords pour un acheminement et une évacuation d'un sang à purifier au moyen du dispositif de dialyse (2).

11. Dispositif de dialyse (2) selon l'une des revendications précédentes, **caractérisé en ce que** le liquide de dialyse est acheminé à la membrane de dialyse (8) par le circuit d'acheminement (4), le dialysat est évacué de la membrane de dialyse (8) par le circuit d'évacuation (10) ; et que le liquide de dialyse acheminé à la membrane de dialyse (8) est tempéré avec la pompe à chaleur (12).

12. Dispositif de dialyse (2) selon l'une des revendications précédentes, **caractérisé en ce que** dans un premier mode de fonctionnement, le liquide de dialyse acheminé à la membrane de dialyse (8) est chauffé grâce à la pompe à chaleur (12).

13. Dispositif de dialyse (2) selon la revendication 12, **caractérisé en ce que** le circuit d'acheminement (4) et le circuit d'évacuation sont nettoyés au moyen d'un liquide de nettoyage parcourant le circuit d'acheminement (4) et le circuit d'évacuation et ensuite le dispositif de dialyse (2) est rincé, dans lequel, pendant le rinçage, dans un deuxième mode de fonctionnement, un liquide de rinçage ou le liquide de dialyse est refroidi dans le circuit d'acheminement (4) au moyen de la pompe à chaleur (12).
